# EUROPEAN PATENT APPLICATION

(11) **EP 2 617 716 A1**
(43) Date of publication of application: **24.07.2013**
(21) Application number: 12151646.2
(22) Date of filing: 18.01.2012
(51) Int. Cl.: C07D 235/16

(54) **Process for the preparation of bendamustine hydrochloride and related compounds**

(71) Applicant: Arevipharma GmbH, 01445 Radebeul (DE)
(72) Inventor: Schickaneder, Helmut, 90542 Eckental (DE); Schickaneder, Christian, 91207 Lauf a. d. Pegnitz (DE); Limmert, Michael, 01277 Dresden (DE)
(74) Representative: Prüfer & Partner GbR European Patent Attorneys

(57) **Abstract**

Disclosed are processes for chlorination of the dihydroxy bendamustine precursor and the purification of bendamustine and related compounds.

## Description

### Field of the invention

The present invention relates to a process for preparing bendamustine hydrochloride, derivatives and related compounds thereof.

### Background of the invention

### Bendamustine having the structural formula

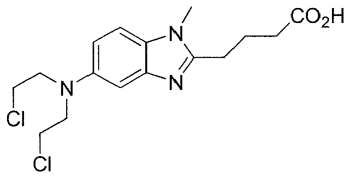

is a nitrogen mustard belonging to the family of drugs called alkylating agents. Bendamustine has been shown to be effective in the treatment of chronic lymphocytic leukemias and lymphomas. Bendamustine is normally used in its hydrochloride salt form as active agent.

CN 10169359 A discloses a process for preparing bendamustine hydrochloride in which dihydroxy bendamustine ethyl ester 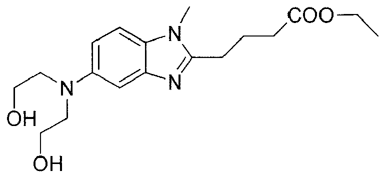 is chlorinated using POCl₃ as chlorinating agent in the presence of an organic solvent selected from the group consisting of toluene, xylene, dichloromethane and chloroform. The crude product of the chlorination reaction in form of bendamustine ethyl ester 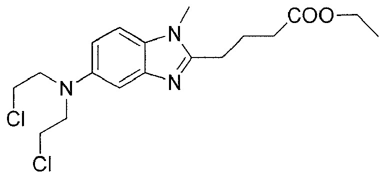 is cleaved by refluxing in concentrated hydrochloric acid in the presence of activated charcoal for a relative long time of 4 hours. Then, water is added to the resulting reaction mixture in order to precipitate crude bendamustine in the form of its hydrochloride salt. Finally, the crude bendamustine hydrochloride is recrystallized from a mixture of THF/water.

WO 2010/042568 A1 discloses a process for preparing bendamustine hydrochloride in which chlorination of dihydroxy bendamustine methyl ester is performed by reacting said ester with 2-chloro acetic acid in the presence of borane-tetrahydrofurane, and subsequently, both ester cleavage and conversion to the hydrochloric salt of bendamustine are carried out by treating with concentrated hydrochloric acid, as shown in Scheme 1 below.

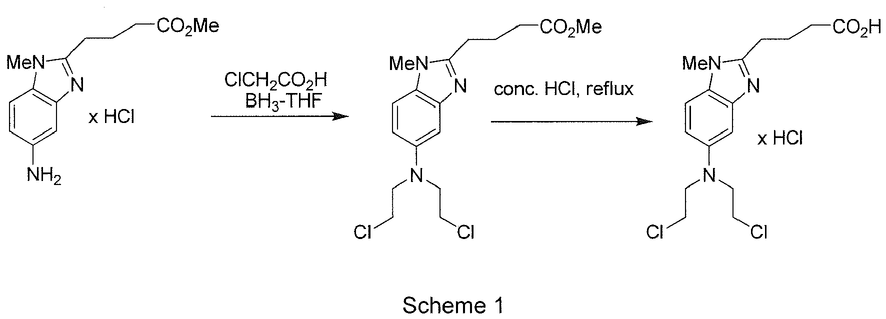

Furthermore, WO 2009/120386 A2 discloses a process for preparing bendamustine hydrochloride in which chlorination of dihydroxy bendamustine diethyl ester is performed by reacting said ester with thionylchloride in chloroform as the solvent, and subsequently, both ester cleavage and conversion to the hydrochloric salt of bendamustine are carried out by treating with concentrated hydrochloric acid, as shown in Scheme 2 below.

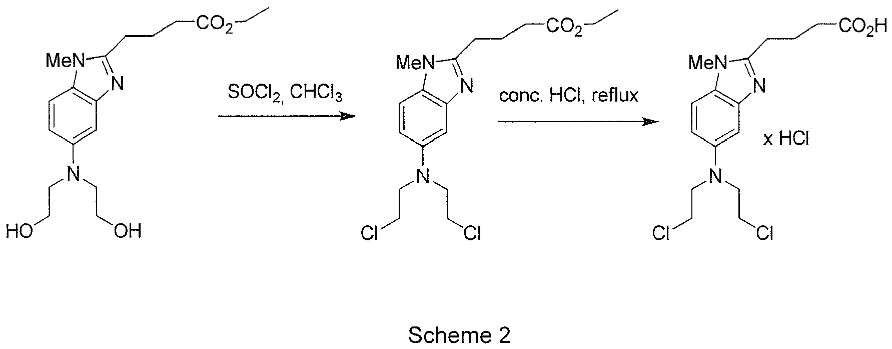

The object of the present invention is to provide an improved process for preparing bendamustine hydrochloride which is suitably adapted for obtaining bendamustine hydrochloride, derivative or related compounds thereof.

### Summary of the invention

Various aspects, advantageous features and preferred embodiments of the present invention as summarized in the following items, respectively alone or in combination, contribute to solving the object of the invention:
(1) A process for preparing a compound of formula II 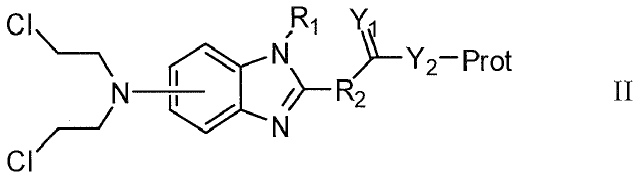 , wherein R₁ is alkyl, aryl or alkylaryl, R₂ is alkylene, arylene, alkylarylene or arylalkylene, Y₁ and Y₂ independently from each other represent oxygen or sulphur, and Prot is a protective group for carboxylic acids, thiol acids (-COSH), thion acids (-CSOH) and -CSSH acids,
   , in which process a compound of formula I 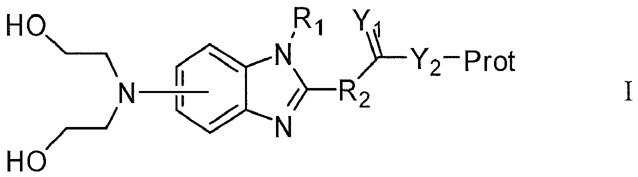 , wherein R₁, R₂, Prot, Y₁ and Y₂ are defined as above,
   is added portionwise without solvent to phosphorous oxychloride (POCl₃) in order to obtain the compound of formula 11 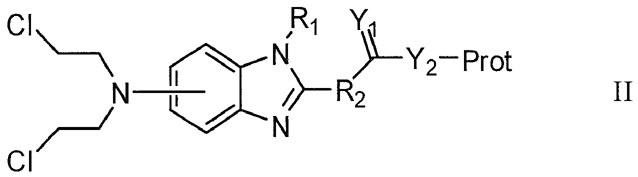 , wherein R₁, R₂, Prot, Y₁ and Y₂ are defined as above.
   The term "alkyl(ene)" as used herein means straight, branched or cyclic hydrocarbons of 1 to 12 carbon atoms, preferably 1 to 8 carbon atoms and more preferably 1 to 6 carbon atoms.
   The term "aryl(ene)" as used herein means hydrocarbon aryls of 3 to 12 carbon atoms, preferably single or condensed six-membered rings, more preferably phenyl.
   The term "alkylaryl(ene)" an "arylalkylene" as used herein means that the aforementioned aryl(ene) moieties are incorporated into the aforementioned straight or branched alkyl(ene) moieties either at one of the proximal or distal ends of the alkyl(ene) chain or between the alkyl(ene) chains. For example, for R₁, proximal end means adjacent to the nitrogen atom of the benzimidazole ring of compound of formula II, while distal means the terminal carbon of the alkyl or aryl moiety which is furthermost from said nitrogen atom. For R₂ proximal end means adjacent to -CY₁- of the -CY₁-Y₂- ester group of compound of formula II, while distal means the terminal carbon of the alkyl(ene) moiety which is furthermost from said -CY₁- moiety.
   The term "protective group for carboxylic acids, thiol acids (-COSH), thion acids (-CSOH) and -CSSH acids" as used herein means any group known in the art which can be used for protecting a -CY₁-Y₂-H acid moiety, with the proviso that said protective group is stable under the conditions applied for converting compound of formula I to compound of formula II. On the other hand, said protective group can be readily removed from compound of formula II in a subsequent reaction step under cleavage conditions which will not adversely affect the structure of compound of formula II. For example, the protective group may be represented by methyl, ethyl, propyl, butyl, 2,2,2-trichloroethyl, trimethylsilyl or t-butyldimethylsilyl. Methyl, ethyl, propyl and butyl represent particularly suitably protecting groups, since they can be formed by transesterification of a -CY₁-Y₂-H acid moiety with readily available C₁-C₄ alkyl alcohols.
   The bis-(2-chloroethyl)amino-group located at the benzimidazole ring structure of compounds of formulae I and II can be located at any one of positions 4, 5, 6 or 7 of the benzimidazole ring structure. Preferably, the bis-(2-chloroethyl)amino-group is located at the 5 position of the benzimidazole ring structure, as exemplary illustrated below for compound of formula II: 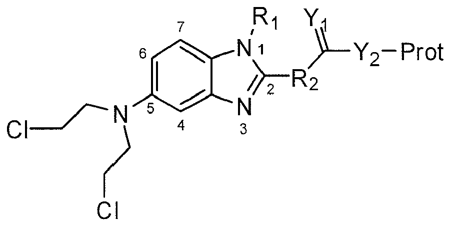 Preferably, the portionwise addition is carried out in that small portions of the solid compound of formula I, without dilution in solvent, are added to POCl₃ such that the temperature of the provided POCl₃ does not significantly rise (e.g. not more than about 10°C per portionwise addition) due to exothermic reaction of ester and POCl₃.
   The procedural concept according to this aspect of the invention provides for compound of formula II having exceptional high purity owing to the simple and efficient reaction control. In particular, the combination of the order of addition, namely adding solid compound of formula I to POCl₃, and the addition in a portionwise manner render it possible to advantageously control the exothermic reaction. That is, overheating due to exothermic reaction can be avoided, which in turn provides for both improved operational safety and significantly higher purity of the product, since less decomposition products will be formed under these controlled reaction conditions.
(2) The process according to item (1), wherein the reaction is carried out in the absence of a solvent.
   According to this embodiment, it can be dispensed with removal of a solvent by distillation and a longer lifetime of the production line is provided for, since in such distillative removal of a solvent, highly corrosive gaseous POCl₃ would corrode parts of the production line which are in contact with the gaseous phase.
(3) The process according to item (1) or (2), wherein 5 to 9 molar equivalents of POCl₃ are used relative to the molar amount of compound of formula I, preferably 7 to 8 molar equivalents POCl₃.
   This preferred embodiment provides for improvement of environmental friendliness of the process, while there is also an improvement in view of the working conditions, since the amount of corrosive and toxic POCl₃ is significantly reduced compared to conventional conditions for chlorinating compound of formula I using POCl₃ as the chlorinating agent. For example, in the chlorination step of CN 101691359 A, the amount of POCl₃ is about 10 to 30 equivalents relative to 4-{5-[bis-(2-chloroethyl)amino]-1-methyl-1H-benzoimidazol-2-yl}-butanoic acid ethyl ester.
(4) The process according to any one of items (1) to (3), wherein the POCl₃ is heated to about 60-70°C before addition of the compound of formula I.
(5) The process according to any one of items (1) to (4), wherein subsequent to a complete addition of compound of formula I, the resulting reaction mixture is heated to reflux temperature for a predetermined time, preferably for 10 to 30 min, more preferably 15 to 25 min.
   According to the preferred embodiments of items (4) and (5), the reactions conditions are suitably selected in terms of reaction temperature and reaction time in order to provide mild reaction conditions. Thus, a stable and reliably process is provided, since less decomposition products are formed, and furthermore, substantial amounts of energy are saved owing to the relatively short reaction times.
(6) The process according to item (5), wherein subsequent to refluxing of the reaction mixture, the resulting reaction mixture is cooled to room temperature.
(7) A process for preparing the compound of formula II 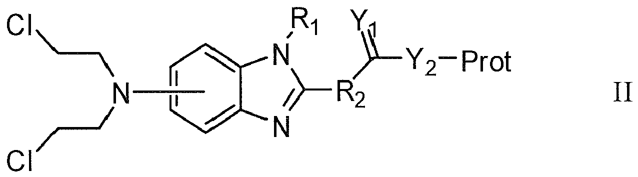 , wherein R₁ is alkyl, aryl or alkylaryl, R₂ is alkylene, arylene, alkylarylene or arylalkylene, Y₁ and Y₂ independently from each other represent oxygen or sulphur, and Prot is a protective group for carboxylic acids, thiol acids (-COSH), thion acids
   (-CSOH) and -CSSH acids
   , wherein subsequent to a conversion of the compound of formula I 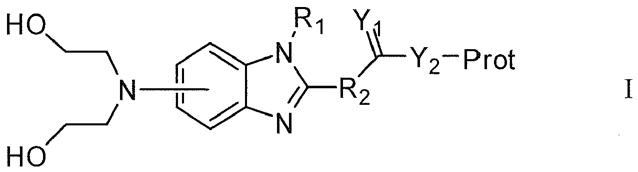 , wherein R₁, R₂, Prot, Y₁ and Y₂ are defined as above, to compound of formula II 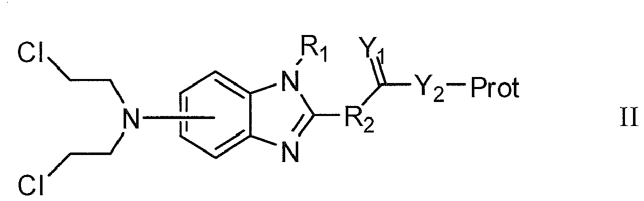 , wherein R₁, R₂, Prot, Y₁ and Y₂ are defined as above,
   by means of a chlorinating agent, a solubilizer is added to the resulting reaction mixture, which reaction mixture is then added portionwise to an aqueous solution of an inorganic proton acceptor.
   The terms "alkyl", "aryl" and "alkylaryl" are defined as above.
   As to the position of the bis-(2-chloroethyl)amino-group located at the benzimidazole ring structure of compounds of formulae I and II, reference is made to the explanations under item (1) above.
   The term "solubilizer" as used herein means a chemical compound which provides for homogeneous admixing of water and organic reaction mixture. That is, by means of the solubilizer, the reaction mixture comprising water and the organic reaction mixture of the chlorination is in the form of a single-phase.
   The term "inorganic proton acceptor" as used herein means a Brønsted base which provides for accepting protons of acids formed during the chlorination reaction and hydrolysis of the residual chlorinating reagent. Such inorganic proton acceptor should not have a too strong base strength in order to avoid cleavage of the carboxylic ester group. Preferably, the inorganic proton acceptor is selected from the group consisting of alkaline metal carbonates and alkaline metal hydrogencarbonates, more preferably Na₂CO₃, NaHCO₃, K₂CO₃ and KHCO₃.
   The term "portionswise" as used herein means that portions of the reaction mixtures in form of a liquid or suspension are added to the aqueous solution of an inorganic proton acceptor in the form of drops or in higher amounts. The size of the portions added is advantageously adapted to the size of the reaction batch and the kind of inorganic base used. For example, in the particular embodiment where alkaline metal carbonates or alkaline metal hydrogencarbonates are used as inorganic proton acceptor, the size of the portions of reaction mixture which are added is selected in a way that the amount of CO₂-foam formed is advantageously limited in order to avoid a foaming over in the reaction vessel.
   According to this preferred aspect of the invention, a process for preparing compound of formula II is provided in which application of a solubilizer provides for significantly improved purity of the product, since said solubilizer renders possible precipitation of the compound of formula II in a single water/organic phase. It was surprisingly found by the present inventors that said solubilizer renders possible to generate a solid precipitate of the respective compound of formula II, in which form compound of formula II was efficiently prevented from hydrolyzing when coming into contact with water. Thereby, the formation of the hydrolysis products, namely the respective monohydroxy and dihydroxy derivatives of formulae 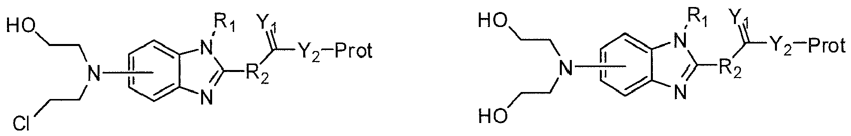 is efficiently prevented. In turn, purification of the precipitated compound of formula II is significantly simplified, since there is no need to remove the aforementioned hydrolysis products, which are difficult to remove by purification from the desired compound of formula II.
(8) The process according to item (7), wherein the chlorinating agent is POCl₃, preferably, chlorination by means of POCl₃ is carried out by applying a process according to any of items (1) to (6).
(9) The process according to item (7) or (8), wherein the solubilizer is an end-capped ethylene glycol or polyethyleneglycol of formula

   A-(CH₂-CH₂)ₙ-B

   , wherein n = 1 to 30 and A and B independently from each other represent C1-C12 alkyl, preferably n = 1 to 20 and A and B independently from each other represent C1-C8 alkyl, even more preferably n = 1 to 10 and A and B independently from each other represent C1-C4 alkyl, yet even more preferably n = 1 to 10 and A and B represent methyl, and in particular 1,2-dimethoxyethane (monoglyme).
   According to this beneficial embodiment of the invention, particularly suitable solubilizers are provided, since they are inert both to POCl₃ and compound of formula 11, that is, they do no react with the aforementioned compounds. It was surprisingly found by the present inventors that these solubilizers provide for a smooth precipitation of the product in solid form, while other solubilizers may lead to the formation of an oily residue instead of the desired solid precipitate and/or discoloration of the product may occur.
(10) The process according to any one of items (7) to (9), wherein when the reaction mixture comprising the solubilizer is added to the aqueous solution of an inorganic proton acceptor, the temperature of the aqueous solution is maintained at a temperature between 20 to 30°C.
(11) The process according to any one of items (1) to (10), wherein the compound of formula II is converted to compound of formula III 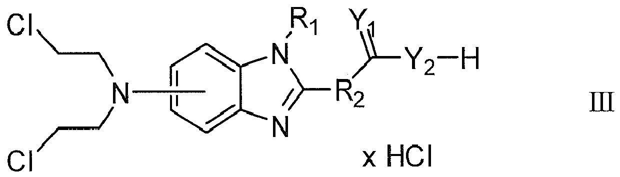 , wherein R₁, R₂, Y₁ and Y₂ are defined as above,
   by means of the steps of:
   a) preparing a mixture of aqueous HCl, crude
      compound of formula II and optionally activated charcoal,
   b) agitating the mixture of step a) for a time interval at room temperature,
   c) optionally separating activated charcoal from the mixture of step b), and
   d) concentrating the reaction mixture.
   The term "room temperature as used herein means a temperature between 15 and 29°C.
(12) The process according to item (11), wherein the aqueous HCl has a concentration of 15 to 32 % by weight relative to the total weight of aqueous HCl, preferably 20 to 32 % by weight, more preferably 25 to 32 % by weight, and most preferably 32 % by weight.
(13) The process according to item (11) or (12), wherein 25 to 50 molar equivalent HCl are used relative to the molar amount of crude compound of formula II, preferably 30 to 40 molar equivalent HCl.
(14) The process according to any one of items (11) to (13), wherein subsequent to step d), the following steps are carried out:
   e) adding the concentrated mixture obtained in step d) to water having room temperature or elevated temperature,
   f) agitating the mixture of step e) for a first time interval during which compound of formula III starts precipitating, and further agitating for a second time interval in order to allow further precipitation of compound of formula III, wherein in case a mixture of step e) having an elevated tempature is applied, the mixture is cooled to room temperature before agitating for the second time interval,
   g) separating the precipitated compound of formula III obtained in step f).
   As to the meaning of the term "room temperature", reference is made to the explanations under item (11) above.
   The term "elevated temperature" means a temperature above room temperature, that is 30°C or more, preferably the elevated temperature is within a range of 35-80°C, preferably 37-70°C, more preferably 40-60°C.
(15) A process for preparing compound of formula III 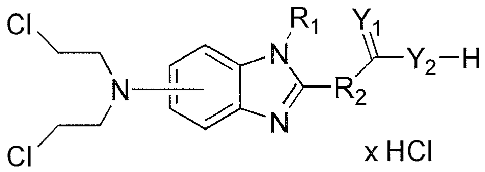 , wherein R₁ is alkyl, aryl or alkylaryl, R₂ is alkylene, arylene, alkylarylene or arylalkylene, and Y₁ and Y₂ independently from each other represent oxygen or sulphur,
   comprising the steps v) to vii):
   v) adding an aqueous solution of crude compound of formula III to water having room temperature or elevated temperature,
   vi) agitating the mixture of step v) for a first time interval during which compound of formula III starts precipitating, and further agitating for a second time interval in order to allow further precipitation of compound of formula III, wherein in case a mixture having an elevated tempature is applied, the mixture is cooled to room temperature before agitating for the second time interval,
   vii) separating the precipitated compound of formula III obtained in step vi), wherein subsequent to step vii), no further recrystallisation in organic solvent is carried out.
   As to the meaning of the term "room temperature", reference is made to the explanations under item (11) above.
   As to the meaning of the term "elevated temperature", reference is made to the explanations uner item (14) above.
   As to the position of the bis-(2-chloroethyl)amino-group located at the benzimidazole ring structure of compound of formula III, the explanations made under item (1) above for compounds of formulae I and III analogously apply for compound of formula III, that is, the bis-(2-chloroethyl)amino-group can be located at position 4, 5, 6 or 7 of the benzimidale ring structure of compound of formula III, wherein position 5 is preferred.
   This aspect of the invention provides for improvement of environmental friendliness of the process, while there is also an improvement in view of the working conditions, since this purification concept dispenses with laborious recrystallisation in a harmful or even toxic organic solvent. Rather, pure compound of formula III is obtained by simple precipitation in water, which is harmless to health and environment.
(16) The process according to any one of items (1) to (15), wherein R₁ is C1-C6 alkyl, R₂ is C1-C6 alkylene, Y₁ and Y₂ represent oxygen, and Prot is C1-C4 alkyl, preferably R₁ is C1-C4 alkyl, R₂ is C1-C4 alkylene, Y₁ and Y₂ represent oxygen, and Prot is C1-C4 alkyl, more preferably R₁ is C1-C3 alkyl, R₂ is C1-C3 alkylene, Y₁ and Y₂ represent oxygen, and Prot is C1-C3 alkyl, yet even more preferably R₁ is methyl, R₂ is propylene, Y₁ and Y₂ represent oxygen, and Prot is methyl or ethyl.
(17) The process according to any one of items (14) to (16), wherein in step f) and step vi) respectively, the first time interval is 1 to 10 min, preferably 2 to 8 min, more preferably 4 to 6 min; and the second time interval is 30 to 120 min, preferably 45 to 90 min and more preferably 50 to 70 min.
(18) The process according to any one of items (15) to (17), wherein prior to steps v) to vii), steps i) to iv) are carried out:
   i) preparing a mixture of aqueous HCl, crude bendamustine hydrochloride and optionally activated charcoal,
   ii) agitating the mixture of step i) for a time interval at room temperature,
   iii) optionally separating activated charcoal from the mixture of step ii), and
   iv) concentrating the reaction mixture.
(19) The process according to any one of items (15) to (18), wherein the aqueous HCl has a concentration of 15 to 32 % by weight relative to the total weight of aqueous HCl, preferably 16 to 25 % by weight, more preferably 18 to 22 % by weight.
(20) The process according to any one of items (15) to (19), wherein 30 to 70 molar equivalent HCl are used relative to the molar amount of crude compound of formula III, preferably 40 to 60 molar equivalent HCl.
   According to the preferred embodiments of item (19) and (20), hydrolysis of the compound of formula III to the respective monohydroxy- and dihydroxy hydrolysis products 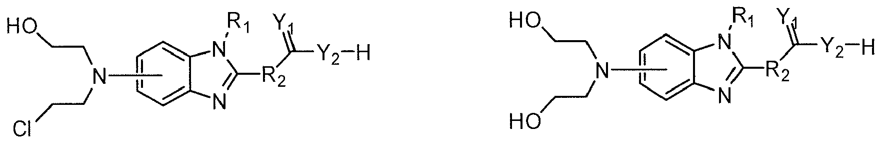 is efficiently prevented. It was surprisingly found by the present inventors that compound of formula III is particularly stable to hydrolysis in a strongly acidic aqueous solution, preferably having a pH of below 2.8, more preferably below 2, while bendamustine hydrochloride partially hydrolyses to the aforementioned monohydroxy- and dihydroxy hydrolysis products at a pH of about 3 to 4. Without whishing to be bound to theory, it is assumed that the strongly acid aqueous solution provides for a precipitation regime which allows for high stability of the precipitated product towards hydrolysis, since the aforementioned critical pH of about 3 to 4 is not reached when the concentrated reaction mixture is added to the water phase. Therefore, purification of the precipitated compound of formula III is significantly simplified, since there is no need to remove the aforementioned hydrolysis products, which are difficult to remove by purification from the desired compound of formula III.
(21) The process according to any one of items (15) to (20), wherein the time interval in step b) and/or ii) is 10 to 30 min, more preferably 15 to 25 min.
(22) The process according to any one of items (15) to (21), wherein in any one of steps c), g), iii) or vii), separation is carried out by means of a filter.
(23) The process according to any one of items (15) to (22), wherein the crude compound of formula III is obtained by a process according to any one of items (1) to (11).
(24) The process according to any one of item (15) to (23), wherein the finally obtained compound of formula III is dried in order to remove water from said hydrochloride and to obtain an anhydrous crystalline form of compound of formula III, preferably, said hydrochloride is dried under reduced pressure.
(25) The process according to item (24), wherein the compound of formula III is dried in the presence of a siccative, preferably said siccative is selected from the group consisting of silica gel, calcium chloride, bentonite and phosphorous pentoxide (P₄O₁₀) more preferably, said siccative is phosphorous pentoxide (P₄O₁₀).
(26) The process according to any one of items (15) to (25), wherein the compound of formula III has a purity of > 99,6% measured by HPLC, preferably > 99.8%, more preferably > 99.9%.
(27) An anhydrous crystalline form of bendamustine hydrochloride 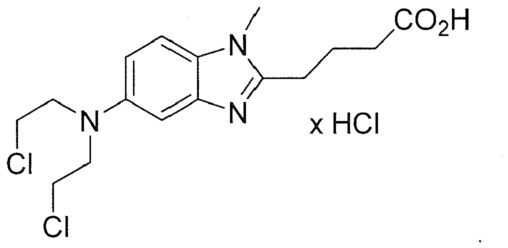 The term "anhydrous" as used herein means that the anhydrous crystalline form of bendamustine hydrochloride may comprise up to 1 wt.-% water (determined by means of Karl Fischer Titration (KFT)) relative to the total molecular weight of the anhydrous crystalline form of bendamustine hydrochloride, preferably up to 0.5 wt.-% water maximally.
(28) The compound according to item (27), which is prepared by a process according to any one of items (1) to (26).
(29) The compound according to item (26) or (27) having a purity of > 99.6% measured by HPLC, preferably > 99.8%, more preferably > 99.9%.
(30) A pharmaceutical composition comprising the anhydrous crystalline form of bendamustine hydrochloride according to any one of items (26) to (29) as a pharmaceutically active agent and at least one pharmaceutically acceptable excipient.
   The term "pharmaceutically active agent" as used herein means any active pharmaceutical ingredient intended for treatment or prophylaxis of a disease of a mammal. In general it means any active pharmaceutical ingredient that has an effect on the physiological conditions of a mammal.
   The term "pharmaceutically acceptable excipient" as used herein means any physiologically inert, pharmacologically inactive material known in the art being compatible with the physical and chemical characteristics of the active agent. Preferably, the pharmaceutically acceptable excipient is selected from the group consisting of binders, disintegrants, bulk polymers and preservatives.
   The term "binder" as used herein means a binding agent which improves adhesion in between particles of the pharmaceutically active agent.
   The term "disintegrant" as used herein means an agent providing for rapid disintegration of a pharmaceutical composition into smaller fragments when in contact with water, wherein dissolution of the pharmaceutical composition and in particular of a pharmaceutically active agent comprised therein is improved.
   The term "bulk polymer" as used herein means a polymeric filling agent which is typically added to a pharmaceutical composition in large amounts, at least in an amount larger than 6% by weight relative to the total weight of the pharmaceutical composition.
   The term "preservatives" as used herein means a substance or mixture of substances which prevents decomposition of a pharmaceutical composition, e.g. microbial or bacterial decomposition.

### Detailed description of the invention

The present invention is now described in more detail by referring to further preferred and further advantageous embodiments and examples, which are however presented for illustrative purposes only and shall not be understood as limiting the scope of the present invention.

According to one aspect of the invention, a general synthetic concept is provided which is particularly suitable for preparing bendamustine-related derivatives. A preferred embodiment of the general synthetic concept of the present invention is illustrated in Scheme 3:

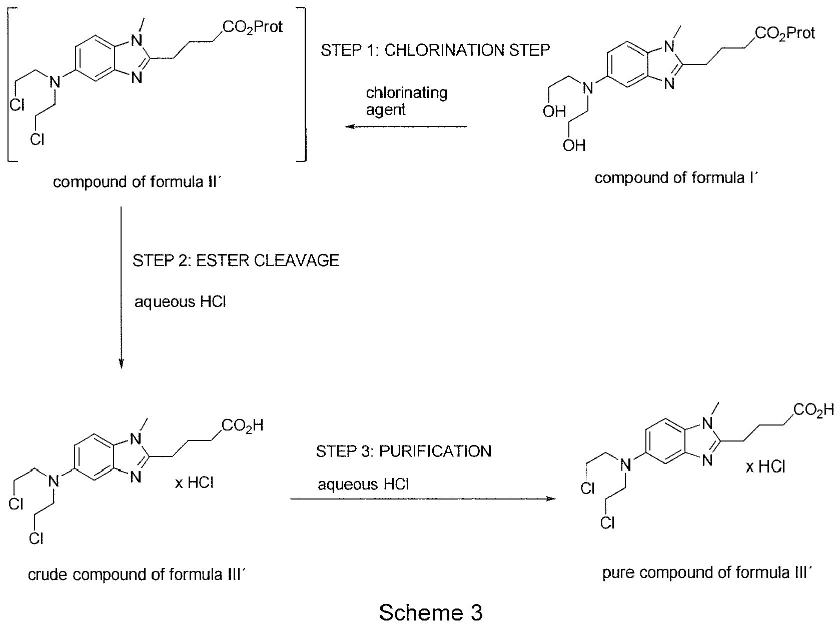

According to the preferred embodiment of Scheme 3 (wherein in compounds of formulae I', II' and III', Prot is defined as in the preceding items, and R₁, R₂, Y₁ and Y₂ of compound of formulae I to III are selected as follows: R₁ = methyl, R₂ = propylene, Y₁ = Y₂ = oxygen), a compound of formula II' is prepared by chlorination of a compound of formula I' using POCl₃ as chlorating agent. Next, compound of formula II' is converted into compound of formula III' by deprotection of the carboxyl moiety under acidic conditions; aqueous HCl is used as a preferred acid. The crude compound of formula III' is purified by recrystallisation in aqueous HCl. Finally, in order to obtain an anhydrous crystalline form of bendamustine hydrochloride, compound of formula III' is dried in order to remove water adsorbtively bound to compound of formula III'. It is understood that the process depicted in Scheme 3 is also applicable to compounds of formulae I to III having other substituents R₁, R₂, Y₁ and Y₂ than the exemplary compounds of formulae I' to III' shown in Scheme 3.

Compounds of formula I' are readily available. For example, a compound of formula I' in which Prot is ethyl can be readily prepared by reacting 4-[(2,4-dinitro-phenyl)-methyl-caramoyl]-butyric acid ethyl ester with ethylene oxide and acetic acid as described in e.g. DD 34727.

According to one aspect of the invention, a chlorination step (cf. STEP 1 in Scheme 3) for preparing compound of formula II can be carried out by means of two alternative process variants A and/or B, which alone or in combination provide for a compound of formula II having exceptional high purity:

In process variant A, a compound of formula I 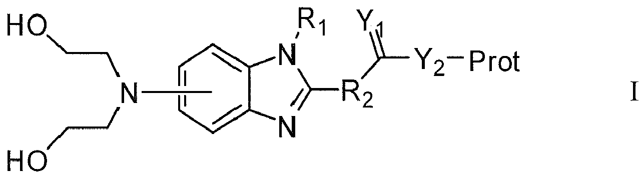 , wherein R₁ is alkyl, aryl or alkylaryl, R₂ is alkylene, arylene, alkylarylene or arylalkylene, Y₁ and Y₂ independently from each other represent oxygen or sulphur, and Prot is a protective group for carboxylic acids, thiol acids (-COSH), thion acids (-CSOH) and - CSSH acids,
is added portionwise without solvent to phosphorous oxychloride (POCl₃) in order to obtain the compound of formula II 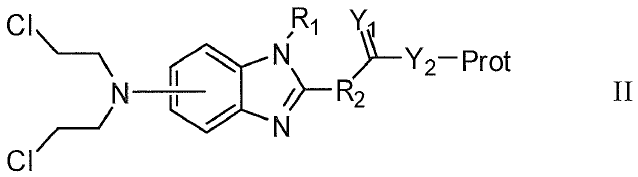 , wherein R₁, R₂, Prot, Y₁ and Y₂ are defined as above.

In process variant B, a compound of formula I 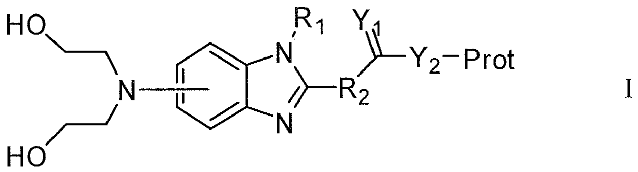 , wherein R₁, R₂, Prot, Y₁ and Y₂ are defined as above,
is converted to a compound of formula II 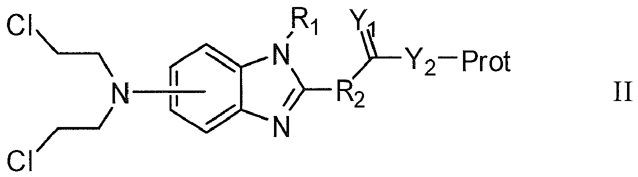 , wherein R₁, R₂, Prot, Y₁ and Y₂ are defined as above,
by means of a chlorinating agent, wherein a solubilizer is added to the resulting reaction mixture, which reaction mixture is then added portionswise to an aqueous solution of an inorganic proton acceptor.

Process variants A and B respectively represent alternative processes for preparing a compound of formula II which processes provide for a simple and efficient carrying out of a chlorination reaction, while although, an exceptional high product purity is achieved. In particular, both process variants provide the possibility of getting the new anhydrous crystalline form of bendamustine hydrochloride and optionally related compound forms.

In particular, the procedural concept of process variant A provides for a simple and efficient reaction control by paying attention to the both the order of addition, namely adding solid compound of formula I to POCl₃, and the addition in a portionwise manner. Thereby, it is rendered possible to advantageously control the exothermic reaction, and thus, overheating due to exothermic reaction can be avoided. This in turn provides for both improved operational safety and significantly higher purity of the product, since less decomposition products will be formed under these controlled reaction conditions.

As to the procedural concept of process variant B, the application of a solubilizer renders possible precipitation of the compound of formula II in a single water/organic phase. Thereby, it was surprisingly found by the present inventors that said solubilizer renders possible to generate a solid precipitate of the respective compound of formula II, in which form compound of formula II was efficiently prevented from hydrolyzing when coming into contact with water. Hence, the formation of the hydrolysis products, namely the respective monohydroxy and dihydroxy derivatives of formulae 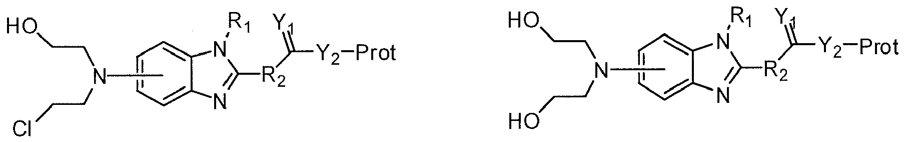 which are difficult to remove by purification, is efficiently prevented. This in turn provides for a significantly simplified purification of the thus obtained compound of formula II.

Preferably, in process variant A, the chlorination reaction is carried out in the absence of a solvent. In this way, it can be dispensed with removal of a solvent by distillation. In addition, a longer lifetime of the production line is provided for, since in such distillative removal of a solvent, highly corrosive gaseous POCl₃ would corrode parts of the production line which are in contact with the gaseous phase.

According to another embodiment of the invention, in process variant A, 5 to 9 molar equivalents of POCl₃ are used relative to the molar amount of compound of formula I, preferably 7 to 8 molar equivalents POCl₃. The aforementioned molar amounts of POCl₃ provide for an improvement of environmental friendliness of the process, since the amount of POCl₃ is substantially reduced compared to conventional chlorination processs applying POCl₃. Furthermore, there is also an improvement in view of the working conditions, since the amount of corrosive and toxic POCl₃ is significantly reduced compared to conventional conditions for chlorinating compound of formula I using POCl₃ as the chlorinating agent. E.g., in the chlorination step of CN 101691359 A, the amount of POCl₃ is about 10 to 30 equivalents relative to 4-{5-[bis-(2-chloroethyl)amino]-1-methyl-1H-benzoimidazol-2-yl}-butanoic acid ethyl ester.

According to still another preferred embodiment, in process variant A, the POCl₃ is heated to about 60-70°C before addition of the compound of formula I. Furthermore, according to yet another embodiment, in process variant A, subsequent to a complete addition of compound of formula I, the resulting reaction mixture is heated to reflux temperature for a predetermined time, preferably for 10 to 30 min, more preferably 15 to 25 min. In the two aforementioned embodiments, the reactions conditions are suitably selected in terms of reaction temperature and reaction time in order to provide mild reaction conditions. Thereby, a stable and reliably process is provided, since less decomposition products are formed. Furthermore, substantial amounts of energy are saved owing to the relatively short reaction times.

In a particularly preferred embodiment of process variant B, the solubilizer is an end-capped ethylene glycol or polyethyleneglycol of formula

A-(CH₂-CH₂)ₙ-B

,wherein n = 1 to 30 and A and B independently from each other represent C1-C12 alkyl, preferably n = 1 to 20 and A and B independently from each other represent C1-C8 alkyl, even more preferably n = 1 to 10 and A and B independently from each other represent C1-C4 alkyl, yet even more preferably n = 1 to 10 and A and B represent methyl, and in particular 1,2-dimethoxyethane (monoglyme). In this way, particularly suitable solubilizers are provided, since they are inert both to POCl₃ and compound of formula II, that is, they do no react with the aforementioned compounds. In particular, it was surprisingly found by the present inventors that these solubilizers provide for a smooth precipitation of the product in solid form, while other solubilizers may lead to the formation of an oily residue instead of the desired solid precipitate and/or discoloration of the product may occur.

Preferably, process variant A and process variant B are combined. Thereby, the aforediscussed advantages of both procedural concepts are utilized in a single chlorination step.

According to a preferred embodiment of the invention, an ester cleavage step (cf. STEP 2 in Scheme 3) for preparing crude compound of formula III 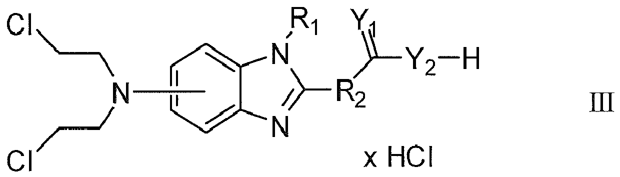 , wherein R₁ is alkyl, aryl or alkylaryl, R₂ is alkylene, arylene, alkylarylene or arylalkylene,
and Y₁ and Y₂ independently from each other represent oxygen or sulphur,
is carried out by a process comprising the following steps:
a) preparing a mixture of aqueous HCl, crude compound of formula II 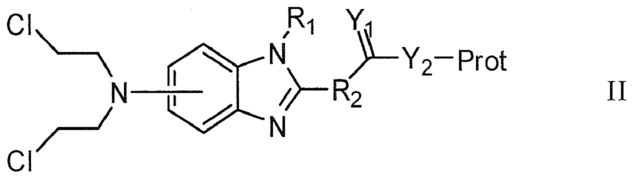 , wherein R₁ is alkyl, aryl or alkylaryl, R₂ is alkylene, arylene, alkylarylene or arylalkylene, Y₁ and Y₂ independently from each other represent oxygen or sulphur, and Prot is a protective group for carboxylic acids, thiol acids (-COSH), thion acids (-CSOH) and -CSSH acids,
   and optionally activated charcoal,
b) agitating the mixture of step a) for a time interval at room temperature,
c) optionally separating activated charcoal from the mixture of step b), and
d) concentrating the reaction mixture.

In this way, and by performing step b) for a suitable time, the -CY₁-Y₂- moiety of compound of formula II is readily deprotected under mild conditions, wherein the use of HCl as deprotecting agent renders possible to form a pharmaceutically acceptable salt in form of compound of formula III already within the ester cleavage step.

According to a further aspect of the invention, a purification process (cf. STEP 3 in Scheme 3) for obtaining pure compound of formula III, 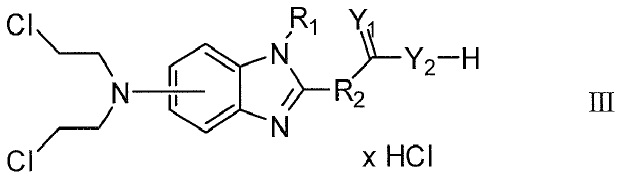 , wherein R₁ is alkyl, aryl or alkylaryl, R₂ is alkylene, arylene, alkylarylene or arylalkylene, and Y₁ and Y₂ independently from each other represent oxygen or sulphur,
is carried out by a process comprising the following steps v) to vii):
v) adding an aqueous solution of crude compound of formula III to water having room temperature or elevated temperature,
vi) agitating the mixture of step v) for a first time interval during which compound of formula III starts precipitating, and further agitating for a second time interval in order to allow further precipitation of compound of formula III, wherein in case a mixture having an elevated tempature is applied, the mixture is cooled to room temperature before agitating for the second time interval,
vii) separating the precipitated bendamustine obtained in step vi),
wherein subsequent to step vii), no further recrystallisation in organic solvent is carried out.

This purification procedure renders possible to dispense with laborious recrystallisation in a harmful or even toxic organic solvent, and thus, environmental friendliness of the process is improved, while there is also an improvement in view of the working conditions. By means of simple precipitation in water, which is harmless to health and environment, pure compound of formula III is obtained. In particular, since no further recrystallisation in organic solvent is carried out, advantageously, the obtained crystalline product is free of organic solvent(s), in particular, the crystalline product is free of organic solvent(s) incorporated in the crystal lattice of the product. That is, the formation of a solvate form comprising organic solvent(s) is effectively prevented.

The first and second time intervals can be suitably adjusted and predetermined in order to achieve a substantially quantitative precipitation of compound of formula III. Preferably, the first time interval is ended when first visible amounts of compound of formula III precipitate. In order to improve and accelerate precipitation, it is preferable to cool the precipitation mixture to "room temperature" within a second time interval. However, in case precipitation works smooth and the aforementioned cooling does not seem to be necessary, the precipitation mixture is simply agitated for a total time interval being the sum of the first and second time interval.

Preferably, in the above described purification process, the aqueous HCl has a concentration of 15 to 32 % by weight relative to the total weight of aqueous HCl, more preferably 16 to 25 % by weight, even more preferably 18 to 22 % by weight. Furthermore, according to another preferred embodiment, 30 to 70 molar equivalent HCl are used relative to the molar amount of crude compound of formula III, more preferably 40 to 60 molar equivalent HCl. The two aforementioned preferred embodiments alone or in combination provide for an efficient prevention of the hydrolysis of the compound of formula III to the respective monohydroxy- and dihydroxy hydrolysis products

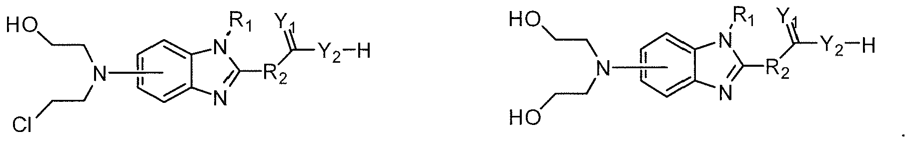

It was surprisingly found by the present inventors that compound of formula III is particularly stable to hydrolysis in a strongly acidic aqueous solution, preferably having a pH of below 2.8, even more at below 2, whereas bendamustine hydrochloride partially hydrolyses to the aforementioned monohydroxy- and dihydroxy hydrolysis products at a pH of about 3 to 4. Without whishing to be bound to theory, it is assumed that the strongly acid aqueous solution provides for a precipitation regime which allows for high stability of the precipitated product towards hydrolysis, since the aforementioned critical pH of about 3 to 4 is not reached when the concentrated reaction mixture is added to the water phase. Hence purification of the precipitated compound of formula III is significantly simplified, since there is no need to remove the aforementioned hydrolysis products, which are difficult to remove by purification from the desired compound of formula III.

According to a another preferred embodiment of the invention, compound of formula III prepared according to the above described purification process is dried in order to remove water from said hydrochloride typically comprising about 5 wt-% water and to obtain an anhydrous crystalline form of compound of formula III. Preferably, drying is carried out by applying reduced pressure, optionally in combination with the application of a siccative.

Surprisingly, even though compound of formula III obtained in the above described purification process provisionally typically contains about 5 wt-% of water relative to the total molecular weight of compound of formula III and thus on a first sight seems to represent a monohydrate, water can be removed from the compound such that an anhydrous product is obtained having a content of residual water of only up to 1 wt-% water relative to the total molecular weight of compound of formula III, preferably less than 0.5 wt-% of water. By contrast, in "true monohydrates", the water would be substantially regularly incorporated into the crystal lattice of an organic or inorganic compound, and hence, the water as a part of the crystal lattice could not have been removed by further drying of the compound such as vacuum drying.

Even more surprisingly, it was found that the aforedescribed drying step allows to obtain an anhydrous crystalline form of bendamustine hydrochloride

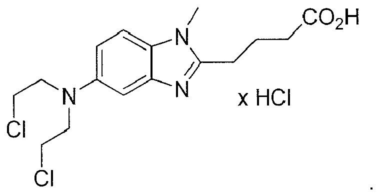

Owing to its (substantially) water freeness, the obtainable anhydrous crystalline form of bendamustine hydrochloride typically provides for a substantially improved shelf life stability compared to water containing forms of bendamustine hydrochloride such as hydrates, since bendamustine is an alkylating agent (also called "alkyl-lost") which readily decomposes in the presence of water. The following examples are merely illustrative of the present invention and they should not be considered as limiting the scope of the invention in any way. The examples and modifications or other equivalents thereof will become apparent to those versed in the art in the light of the present entire disclosure.

### Examples

### Analytical methods

### Water (Karl Fischer titration (KFT))

### According to Ph.Eur., 2.5.12 (method A); Titrando 835 (Metrohm); platinum electrode (Fa. Metrohm)

| **HPLC / MS** | |
|---|---|
| Instrumentation: | |
| | - P680, ASI 100, TCC 100, PDA 100 (Dionex) |
| | - Surveyor MSQ (Thermo Electron Corporation) |
| Test solution: | (c = 1 mg/ml), solvent: acetonitrile/methanol = 8/2 (v/v). |
| Column: | Merck LiChrospher 100 DIOL, 5µm, (250 x 4,0) mm |
| Mobile phase A: | acetonitrile/methanol = 9/1 (v/v) + 0.5 g/L ammonium acetate + 1.4 mL/L acetic acid. |
| Mobile phase B: | acetonitrile/methanol = 6/4 (v/v) + 0.5 g/L ammonium acetate + 1.4 mL/L acetic acid. |
| Gradient: | 0 min 100% A; 10 min 100% A; 15 min 10% A; 20 min 10% A; 22 min 100% A; 28 min 100% A. |
| Injection: | 10 µL |
| Flow rate: | 1.3 mL/min |
| **Mass spectrometry** | ESI+ |
| **NMR** | |
| Instrumentation: | Bruker Avance, 600 MHz, Temperature 295 K |
| | Bruker Avance, 500 MHz, Temperature 295 K |

### Example 1: Preparation of compounds of formula II

### a) Ethyl ester of bendamustine

A 500 mL three-necked round bottom flask equipped with a magnetic stirring bar, internal thermometer and a reflux-condenser with oil ventile was charged with phosphorus oxychloride (134 g, 80.0 mL, 874 mmol) and heated to an internal temperature of about 60-70 °C.

4-{5-[bis-(2-hydroxyethyl)amino]-1-methyl-1H-benzoimidazol-2-yl}-butanoic acid ethyl ester (40 g, 114 mmol) was added in portions. After the addition was completed the mixture was heated to reflux temperature and stirring was continued for a further 15 min. The mixture was allowed to reach room temperature and 1,2-dimethoxyethane (90 mL) was added with stirring (solution 1). A 2000 mL three-necked round bottom flask was charged with potassium bicarbonate (412.1 g, 4115 mmol) and potable water (525 mL). Solution 1 was added slowly with stirring, maintaining an internal temperature of about 20 to 30 °C, after which stirring was continued for a further 60 min. The resulting solid was isolated, washed with water (4 x 100 mL) and used without further purification. The product may optionally be dried in vacuum at temperatures of not more than 40 °C. Yield (moist): 89.26 g; calculated dry: 46.3 g, > 100%; product usually contains residual water even after drying.

¹H NMR (600 MHz, DMSO-d₆, ppm): δ = 7.73 (d, ³J=8.8 Hz, 1H, arom. R₂NCCH=CH), 6.92 (d, ⁴J=2.3 Hz, 1H, arom. R₂NCCH), 6.78 (dd, ³J=8.8 Hz, ⁴J=2.3 Hz, 1H, arom. R₂NCCH=CH), 4.04 (q, ³J=7.1 Hz, 2H, OCH₂CH₃), 3.70 (s, 8H, CH₂CH₂Cl), 3.65 (s, 3H, CH₃N), 2.92 (t, ³J = 7.4 Hz, 2H, CH₂-CH₂-CH₂-COOEt), 2.44 (t, 3J = 7.3 Hz, 2H, CH₂-CH₂-CH₂- COOEt), 2.00 (m, 2H, CH₂-CH₂-CH₂- COOEt).

¹³C-{H}-NMR (150 MHz, DMSO-d₆, ppm): δ = 172.7 (COOEt), 154.4 (arom.), 143.4 (arom.), 142.3 (arom.), 129.3 (arom.), 110.2 (arom.), 110.0 (arom.), 102.3 (arom.), 59.8 (CH₂CH₃), 53.6 (2xCH₂Cl), 41.5 (2xCH₂N), 32.9 (CH₂), 29.4 (CH₃), 25.7(CH₂), 22.2 (CH₂), 14.2 (CH2CH₃).

LC-MS (ESI⁺): m/z = 386.2 (M + H⁺; 100% relative Intensity)

### b) Methyl ester of bendamustine

A 250 mL three-necked round bottom flask equipped with a magnetic stirring bar, internal thermometer and a reflux-condenser with oil ventile was charged with phosphorus oxychloride (101 g, 60.0 mL, 657 mmol) and heated to an internal temperature of about 60 - 70 °C.

4-{5-[bis-(2-hydroxyethyl)amino]-1-methyl-1H-benzoimidazol-2-yl}-butanoic acid methyl ester (30 g, 89.4 mmol) was added in portions. After the addition was completed the mixture was heated to reflux temperature and stirring was continued for a further 15 min. The mixture was allowed to reach room temperature and 1,2-dimethoxyethane (67.5 mL) was added with stirring (solution 1). A 1000 mL three-necked round bottom flask was charged with potassium bicarbonate (321.8 g, 3215 mmol) and potable water (394 mL). Solution 1 was added slowly with stirring, maintaining an internal temperature of about 20 to 30 °C, after which stirring was continued for a further 60 min. The resulting solid was isolated, washed with water (4 x 75 mL) and used without further purification. Yield: 71.6 g, moist.

¹H NMR (500 MHz, DMSO-d₆, ppm): δ = 7.32 (d, ³J=8.8 Hz, 1H, arom. R₂NCCH=CH), 6.93 (d, ⁴J=1.6 Hz, 1H, arom. R₂NCCH), 6.79 (dd, ³J=8.8 Hz, ⁴J=1.6 Hz, 1H, arom. R₂NCCH=CH), 3.71 (s, 8H, CH₂CH₂Cl), 3.65 (s, 3H, CH₃N), 3.60 (s, 3H, CH₃O), 2.83 (t, ³J = 7.4 Hz, 2H, CH₂-CH₂-CH₂-COOMe), 2.49 (t, ³J = 7.3 Hz, 2H, CH₂-CH₂-CH₂-COOMe), 2.02 (m, 2H, CH₂-CH₂-CH₂-COOMe).

¹³C-{H}-NMR (125.77 MHz, DMSO-d₆, ppm): δ = 173.6 (COOMe), 154.8 (arom.), 143.8 (arom.), 142.7 (arom.), 129.8 (arom.), 110.6 (arom.), 110.4 (arom.), 102.7 (arom.), 54.0 (2xCH₂Cl), 51.7 (OCH₃), 41.9 (2xCH₂N), 33.1 (CH₂), 29.8 (CH₃), 26.0(CH₂), 22.5 (CH₂).

### Example 2: Preparation of compound of formula III

### Crude bendamustine hydrochloride

### a) Preparation using the bendamustine ethyl ester of Example 1 a) as the starting material

A 250 mL three-necked round bottom flask equipped with a magnetic stirring bar, internal thermometer and a reflux-condenser with oil ventile was charged with bendamustine ethyl ester (44.6 g moist product, calcd. dry 23 g, 60 mmol (assumed content 100%)), hydrochloric acid (37%, 58 mL), and activated charcoal (1.1 g). The suspension was stirred for about 15 min at temperatures of 25 to 28 °C, filtered and the residue was washed with hydrochloric acid (37%, 2 mL). The combined aqueous solutions were concentrated under reduced pressure. Yet another flask was charged with water (158 mL), which was warmed up to 41 °C and treated with the concentrate as obtained above with vigorous stirring at 41 °C. After the addition was completed the resulting suspension was stirred for 8 min and then cooled to ambient temperature. Stirring was continued for further 30 min and the precipitate was isolated, washed with water (3x 9 mL) and optionally additionally with acetone (3x 9 mL) to yield bendamustine hydrochloride as a colorless solid (20.54 g moist product). Optionally the moist product may be dried at ambient temperature under reduced pressure.

¹H NMR (600 MHz, DMSO-d₆, ppm): δ = 16-14 (s, br, 0.25H, acidic), 13-11 (s, br, 0.5H, acidic), 7.73 (d, ³J=9.2 Hz, 1H, arom. R₂NCCH=CH), 7.11 (dd, ³J=9.2 Hz, ⁴J=2.3 Hz, 1H, arom. R₂NCCH=CH), 6.91 (d, ⁴J=2.3 Hz, 1H, arom. R₂NCCH), 3.90 (s, 3H, CH₃N), 3.83 (t, ³J= 7.0 Hz, 4H, 2xClCH₂), 3.77 (t, ³J = 7.0 Hz, 4H, 2xCH₂N), 3.18 (t, ³J = 7.6 Hz, 2H, CH₂-CH₂-CH₂-COOEt), 2.43 (t, 3J = 7.3 Hz, 2H, CH₂-CH₂-CH₂-COOEt), 2.03 (m, 2H, CH₂-CH₂-CH₂-COOEt); acidic protons diminished in intensity, presumably due to solvent exchange.

¹³C-{H}-NMR (150 MHz, DMSO-d₆, ppm): δ = 173.7 (COOH), 151.8 (arom. CN₂), 145.7 (arom.), 131.7 (arom.), 124.8 (arom.), 113.5 (arom.), 112.4 (arom.), 94.7 (arom.), 52.4 (2xCH₂Cl), 41.1 (2xCH₂N), 32.6 (CH₂), 31.0 (CH₃), 24.0 (CH₂), 21.7 (CH₂). HPLC-purity: 100% relative Area

LC-MS (ESI⁺): m/z = 358.1 (M - Cl⁻ - H₂O; 100% relative Intensity)

### b) Preparation using the bendamustine methyl ester of Example 1 b) as the starting material

A 500 mL three-necked round bottom flask equipped with a magnetic stirring bar, internal thermometer and a reflux-condenser with oil pressure valve was charged with bendamustine methyl ester (177.93 moist, 92.2 g calc'd. dry, content of dry matter assumed 100%: 248 mmol), hydrochloric acid (37%, 215 mL), and activated charcoal (4.61 g). The suspension was stirred for about 15 min at temperatures of 25 to 28 °C, filtered and the residue was washed with hydrochloric acid (37%, 8 mL). The combined aqueous solutions were concentrated under reduced pressure. Yet another flask was charged with water (585 mL), which was warmed up to 41 °C and treated with the concentrate as obtained above with vigorous stirring at 41 °C. After the addition was completed, the resulting suspension was cooled to ambient temperature within 13 min. Stirring was continued for a further 60 min and the precipitate was isolated, washed with water (3x 45 mL) and optionally additionally with acetone (3 x 45 mL) to yield the title compound as a colourless solid (86.3 g moist product). Optionally the moist product may be dried at ambient temperature under reduced pressure. HPLC-purity: 99.97% relative area.

### Example 3: Purification of compound of formula III

### Pure bendamustine hydrochloride

### a) Purification of the crude bendamustine hydrochloride obtained in Example 2 a)

A three necked round bottom flask equipped with magnetic stirring bar, internal thermometer and a reflux-condenser with oil ventile was charged with crude bendamustine HCl (16.9 g, 40.9 mmol), aqueous hydrochloric acid (20%; 34 mL), and activated charcoal (0.85 g, 70.8 mmol). The suspension was stirred at room temperature for about 15 min and insolubles were removed. The residuals were washed with hydrochloric acid (20%, 2 mL). Water (106 mL) was warmed to 41 °C, and the combined acidic filtrates were added slowly with stirring. Stirring was continued for a further 5 min and the vessel contents were allowed to reach room temperature. Stirring was continued for a further 55 min and the resulting solid was isolated, washed with water (3x 7 mL) and acetone (3x 7 mL). Yield (moist): 15.3 g, Yield (dry) 13.5 g (80%).

¹H NMR (600 MHz, DMSO-d₆, ppm): δ = 16-14 (s, br, 0.40H, acidic), 13-11 (s, br, 0.45H, acidic), 7.73 (d, ³J=9.2 Hz, 1H, arom. R₂NCCH=CH), 7.11 (dd, ³J=9.2 Hz, ⁴J=2.3 Hz, 1H, arom. R₂NCCH=CH), 6.91 (d, ⁴J=2.3 Hz, 1H, arom. R₂NCCH), 3.90 (s, 3H, CH₃N), 3.81 (t, ³J= 6.1 Hz, 4H, 2xClCH₂), 3.77 (t, ³J = 6.1 Hz, 4H, 2xCH₂N), 3.17 (t, ³J = 7.7 Hz, 2H, CH₂-CH₂-CH₂-Ester), 2.41 (t, 3J = 7.3 Hz, 2H, CH₂-CH₂-CH₂-Ester), 2.01 (m, 2H, CH₂-CH₂-CH₂-Ester); acidic protons diminished in intensity, presumably due to solvent exchange.

¹³C-{H}-NMR (150 MHz, DMSO-d₆, ppm): δ = 173.7 (COOH), 151.8 (arom. CN₂), 145.7 (arom.), 131.7 (arom.), 124.8 (arom.), 113.5 (arom.), 112.4 (arom.), 94.7 (arom.), 52.4 (2xCH₂Cl), 41.1 (2xCH₂N), 32.6 (CH₂), 31.0 (CH₃), 24.1 (CH₂), 21.7 (CH₂).
HPLC-purity: 100% relative area
LC-MS: m/z = 358.1 (M⁺ - HCl - H₂O)

Residual water: 4.58% by weight

### b) Purification of the crude bendamustine hydrochloride obtained in Example 2 b)

The purification of the crude bendamustine hydrochloride obtained in Example 2 b) is carried out analogously as described in Example 3 a), wherein crude bendamustine HCl obtained in Example 2 b) is used as the starting material.

### Example 4

### Preparation of an anhydrous crystalline form of bendamustine hydrochloride

Bendamustine hydrochloride obtained in Example 3 a) and b) respectively was dried under reduced pressure, optionally in the presence of phosphorous pentoxide. The content of residual water was 0.22-0.80% by weight relative to the total molecular weight of bendamustine hydrochloride (determined by KFT-titration).

## Claims

1. A process for preparing a compound of formula II 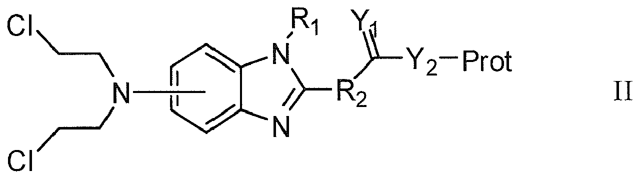 , wherein R₁ is alkyl, aryl or alkylaryl, R₂ is alkylene, arylene, alkylarylene or arylalkylene, Y₁ and Y₂ independently from each other represent oxygen or sulphur, and Prot is a protective group for carboxylic acids, thiol acids (-COSH), thion acids (-CSOH) and -CSSH acids,
, in which process a compound of formula I 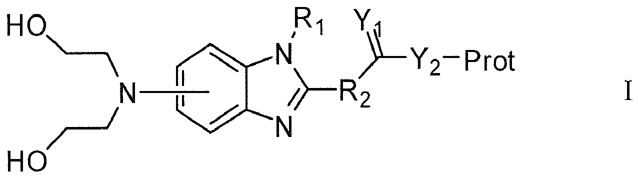 , wherein R₁, R₂, Prot, Y₁ and Y₂ are defined as above,
is added portionwise without solvent to phosphorous oxychloride (POCl₃) in order to obtain the compound of formula II 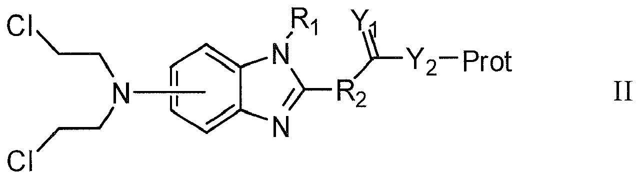 , wherein R₁, R₂, Prot, Y₁ and Y₂ are defined as above.

2. The process according to claim 1, wherein the reaction is carried out in the absence of a solvent.

3. The process according to claim 1 or 2, wherein 5 to 9 molar equivalents of POCl₃ are used relative to the molar amount of compound of formula I, preferably 7 to 8 molar equivalents POCl₃.

4. The process according to any one of claims 1 to 3, wherein the POCl₃ is heated to about 60-70°C before addition of the compound of formula I.

5. The process according to any one of claims 1 to 4, wherein subsequent to a complete addition of compound of formula I, the resulting reaction mixture is heated to reflux temperature for a predetermined time, preferably for 10 to 30 min, more preferably 15 to 25 min.

6. A process for preparing the compound of formula II 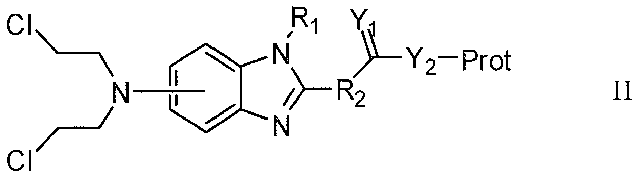 , wherein R₁ is alkyl, aryl or alkylaryl, R₂ is alkylene, arylene, alkylarylene or arylalkylene, Y₁ and Y₂ independently from each other represent oxygen or sulphur, and Prot is a protective group for carboxylic acids, thiol acids (-COSH), thion acids
(-CSOH) and -CSSH acids
, wherein subsequent to a conversion of the compound of formula I 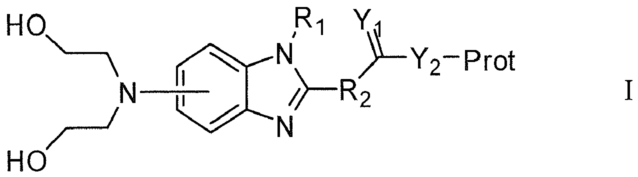 , wherein R₁, R₂, Prot, Y₁ and Y₂ are defined as above, to compound of formula II 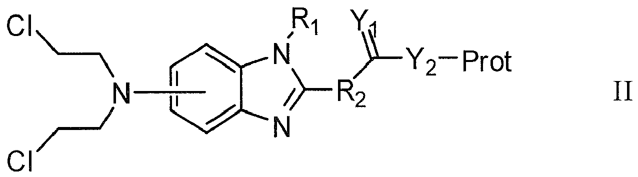 , wherein R₁, R₂, Prot, Y₁ and Y₂ are defined as above,
by means of a chlorinating agent, a solubilizer is added to the resulting reaction mixture, which reaction mixture is then added portionwise to an aqueous solution of an inorganic proton acceptor.

7. The process according to claim 6, wherein the chlorinating agent is POCl_{3;} preferably, chlorination by means of POCl₃ is carried out by applying a process according to any of claims 1 to 5.

8. The process according to claim 6 or 7, wherein the solubilizer is an end-capped ethylene glycol or polyethyleneglycol of formula
A-(CH₂-CH₂)ₙ-B
, wherein n = 1 to 30 and A and B independently from each other represent C1-C12 alkyl, preferably n = 1 to 20 and A and B independently from each other represent C1-C8 alkyl, even more preferably n = 1 to 10 and A and B independently from each other represent C1-C4 alkyl, yet even more preferably n = 1 to 10 and A and B represent methyl, and in particular 1,2-dimethoxyethane (monoglyme).

9. The process according to any one of claims 1 to 8, wherein the compound of formula II is converted to compound of formula III 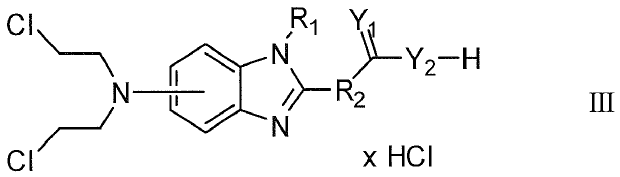 , wherein R₁, R₂, Y₁ and Y₂ are defined as above,
by means of the steps of:
a) preparing a mixture of aqueous HCl, crude
compound of formula II and optionally activated charcoal,
b) agitating the mixture of step a) for a time interval at room temperature,
c) optionally separating activated charcoal from the mixture of step b), and
d) concentrating the reaction mixture.

10. The process according to claim 9, wherein the aqueous HCl has a concentration of 15 to 32 % by weight relative to the total weight of aqueous HCl, preferably 20 to 32 % by weight, more preferably 25 to 32 % by weight, and most preferably 32 % by weight.

11. The process according to claim 9 or 10, wherein 25 to 50 molar equivalent HCl are used relative to the molar amount of crude compound of formula II, preferably 30 to 40 molar equivalent HCl.

12. A process for preparing compound of formula III 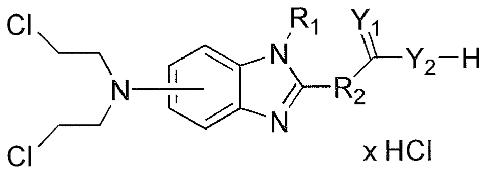 , wherein R₁ is alkyl, aryl or alkylaryl, R₂ is alkylene, arylene, alkylarylene or arylalkylene, and Y₁ and Y₂ independently from each other represent oxygen or sulphur,
comprising the steps v) to vii):
v) adding an aqueous solution of crude compound of formula III to water having room temperature or elevated temperature,
vi) agitating the mixture of step v) for a first time interval during which compound of formula III starts precipitating, and further agitating for a second time interval in order to allow further precipitation of compound of formula III, wherein in case a mixture having an elevated tempature is applied, the mixture is cooled to room temperature before agitating for the second time interval,
vii) separating the precipitated compound of formula III obtained in step vi), wherein subsequent to step vii), no further recrystallisation in organic solvent is carried out.

13. The process according to any one of claims 1 to 12, wherein R₁ is C1-C6 alkyl, R₂ is C1-C6 alkylene, Y₁ and Y₂ represent oxygen, and Prot is C1-C4 alkyl, preferably R₁ is C1-C4 alkyl, R₂ is C1-C4 alkylene, Y₁ and Y₂ represent oxygen, and Prot is C1-C4 alkyl, more preferably R₁ is C1-C3 alkyl, R₂ is C1-C3 alkylene, Y₁ and Y₂ represent oxygen, and Prot is C1-C3 alkyl, yet even more preferably R₁ is methyl, R₂ is propylene, Y₁ and Y₂ represent oxygen, and Prot is methyl or ethyl.

14. The process according to claim 12 or 13, wherein the aqueous HCl has a concentration of 15 to 32 % by weight relative to the total weight of aqueous HCl, preferably 16 to 25 % by weight, more preferably 18 to 22 % by weight.

15. The process according to any one of claims 12 to 14, wherein 30 to 70 molar equivalent HCl are used relative to the molar amount of crude compound of formula III, preferably 40 to 60 molar equivalent HCl.
